# EUROPÄISCHE PATENTSCHRIFT

(11) **EP 2 997 347 B1**
(45) Veröffentlichungstag und Bekanntmachung des Hinweises auf die Patenterteilung: **04.04.2018**
(21) Anmeldenummer: 13742172.3
(22) Anmeldetag: 04.07.2013
(51) Int. Cl.: G01N 3/36, A61F 2/82, F15B 21/12

(54) **ERZEUGUNG VON DRUCKSCHWANKUNGEN IN EINER LEITUNG**
GENERATING PRESSURE FLUCTUATIONS IN A LINE
GÉNÉRATION DE VARIATIONS DE PRESSION DANS UNE CONDUITE

(30) Priorität: 16.05.2013 DE 102013105037
(43) Veröffentlichungstag der Anmeldung: 23.03.2016
(73) Patentinhaber: Eucatech AG, 79618 Rheinfelden (DE)
(72) Erfinder: GIESE, Michael, 79618 Rheinfelden (DE)
(74) Vertreter: Götz, Georg Alois
(86) Internationale Anmeldenummer: PCT/EP2013/064171
(87) Internationale Veröffentlichungsnummer: WO 2014/183809

(56) Entgegenhaltungen:
- DE-A1- 2 305 888
- US-A- 3 202 180
- US-A- 4 250 872
- US-A- 5 670 708

## Beschreibung

Die Erfindung betrifft ein Verfahren und eine Leitungsanordnung mit einer Leitung, bei der Druckschwankungen in einem in der Leitung befindlichen Fluid erzeugbar sind.

In einer Vielzahl von Anwendungsfällen, beispielsweise in US 1965/3202180, DE 1974/2305888 A1 und US 1981/4250872,ist es wünschenswert, Druckschwankungen in einem in einer Leitung befindlichen Fluid zu erzeugen. Der Begriff Leitung steht dabei stellvertretend für eine einzelne Leitung, ein Leitungssystem kommunizierender Leitungen, z.B. ein Röhrensystem oder eine sonstige Hohlkörperanordnung, in welcher ein Fluid vorhanden ist, welches mit Druckschwankungen zu beaufschlagen ist.

Die Erfindung ist auf vielen Gebieten der Technik, z.B. für die Prüfung medizinischer Stents oder für Berstdruckversuche einsetzbar und wird im folgenden lediglich beispielhaft anhand einer Leitungsanordnung in Form einer Testanordnung für medizinische Stents erläutert. Der Oberbegriff Stent schließt dabei Stents, Grafts, deren Kombinationen und ähnliche Gegenstände ein. Bevor Stents in den klinischen Einsatz gelangen, müssen diese ausführlich getestet werden. Insbesondere werden Ermüdungstests durchgeführt, bei welchen die Stents Verformungen ausgesetzt werden.

Eine Testanordnung für Stents ist beispielsweise aus der US 5,670,708 bekannt. Eine radial flexible Leitung, in die der Stent eingesetzt wird, simuliert ein Blutgefäß. Ein Fluid in der Leitung simuliert Blut und Druckpulse im Fluid simulieren die Blutdruckschwankungen durch den Herzschlag des Patienten. Somit erfolgt eine radiale Expansion und Kontraktion der Leitung und damit eine zyklische Verformung des Stents. Druckpulse im Fluid werden durch Expansion und Kompression von Faltenbalgen erzeugt. Die Stents folgen - sich verformend - der radialen Bewegung der Leitung und werden so dem Ermüdungstest unterzogen.

Getestet werden Stents in der Regel für eine Einsatzdauer von zehn Jahren, was bei einer durchschnittlichen menschlichen Herzfrequenz etwa einer Gesamtanzahl von 100 Millionen Druckpulsen entspricht. Nachteilig bei der bekannten Anordnung ist, dass die Faltenbalge bei jedem Druckpuls verformt werden, somit einer hohen Dauerbelastung ausgesetzt sind und z.B. selbst Ermüdungserscheinungen unterliegen.

Aufgabe der vorliegenden Erfindung ist es, ein verbessertes Verfahren zum Erzeugen von Druckschwankungen in einer ein Fluid enthaltenden Leitung und eine verbesserte Leitungsanordnung anzugeben.

Hinsichtlich des Verfahrens wird die Aufgabe gelöst durch ein Verfahren gemäß Patentanspruch 1 zum Erzeugen von Druckschwankungen in einem Fluid, das sich in einer Leitung befindet. Die Leitung ist ein Leitungssystem einer Testanordnung für einen medizinischen Stent, wobei die Leitungsanordnung einen Testabschnitt aufweist, der durch eine Erhöhung des Drucks des Fluids aufweitbar ist. Im Testabschnitt ist der Stent platzierbar oder platziert. Das Verfahren weist folgende Schritte auf:

Die Leitung wird von einem Einlass der Leitung zu deren Auslass hin vom Fluid durchströmt. Am Einlass wird das Fluid fördersteif in den Einlass der Leitung gefördert. "Fördersteif" bedeutet, dass ein konstanter Volumenstrom (Volumen pro Zeiteinheit) des Fluids in den Einlass gefördert wird. Am Auslass wird der Volumenstrom des aus dem Auslass strömenden Fluids zeitlich dynamisch verschieden stark gedrosselt. Die zeitlich dynamische Drosselung erfolgt hierbei insbesondere periodisch. Durch die zeitlich dynamische Änderung der Drosselung, also das verschieden starke Drosseln des ausströmenden Fluids zu verschiedenen Zeitpunkten, werden stromaufwärtig in der Leitung zeitlich dynamische Druckschwankungen, also ein über der Zeit - insbesondere periodisch - variierender Druckverlauf im Fluid in der Leitung erzeugt.

Die Erfindung nutzt damit bewusst das Prinzip des Druckstoßes aus, der in Leitungssystemen in der Regel unerwünscht ist. Ein Druckstoß entsteht stromaufwärts in einer Leitung durch eine stromabwärtige Drosselung eines Fluidstroms in der Leitung, beispielsweise mit Hilfe einer Absperrarmatur. Bei einem Fluid in Form einer Flüssigkeit ist bei gleicher Drosselung der Effekt von Druckstößen stärker - d.h. die Druckschwankungen sind größer - als bei gasförmigen Fluiden, da Flüssigkeiten weniger kompressibel als Gase sind. Die Druckstöße pflanzen sich vom dem Ort der Drosselung, also beispielsweise der Absperrarmatur, stromaufwärts in der Leitung fort.

Als Fluid für den oben genannten Test von Stents wird daher insbesondere Flüssigkeit, als Flüssigkeit insbesondere eine physiologische Kochsalzlösung gewählt. Durch Verwendung einer Flüssigkeit ist der erwünschte Effekt der Druckstöße gegenüber gasförmigen Fluiden vergrößert. Die Kochsalzlösung ist insbesondere zur Stentprüfung geeignet. Das erfindungsgemäße Verfahren bietet aufgrund der Ausnutzung des Druckstoßprinzips den Vorteil, dass zur Drosselung des Fluidstroms bzw. zur veränderbaren Drosselung jedwede geeignete mechanisch arbeitende Drosselvorrichtung verwendet werden kann. Hier sind Ausführungsformen als Ventil, Schieber, Prallplatte usw. denkbar. Diese Vorrichtungen kommen in der Regel ohne elastisch zu verformende Elemente aus und sind daher insbesondere gegenüber bekannten Vorrichtungen mit Faltenbälgen deutlich reduziertem Verschleiß unterworfen. Das Verfahren ist somit verschleißärmer als das bekannte Verfahren durchführbar. Die Förderung des Fluids in den Einlass der Leitung kann ebenfalls durch bekannte, verschleißarme Vorrichtungen erfolgen, wie z.B. durch Verwendung einer pulsationsfreien Pumpe, z.B. einer Zahnradpumpe. Durch den reduzierten Verschleiß der beteiligten Komponenten ist das Verfahren hinsichtlich Ausfallsicherheit und Verfügbarkeit besonders vorteilhaft.

In der Testanordnung ist der aufweitbare Testabschnitt insbesondere zylinderförmig ausgeführt, z.B. in Form eines flexiblen Schlauchs, z.B. eines Silikonschlauchs. Ein Stent kann dort eingebracht und gehalten werden. Der Stent wird gehalten, indem der aufweitbare Testabschnitt einen wenig flexiblen Stent durch eine radial nach innen gerichtete Federkraft hält und/oder der Stent selbst flexibel ausgeführt ist und so radial auswärts federnd im Testabschnitt vorgespannt ist. Insbesondere ist das Leitungssystem mit Ausnahme des Testabschnittes starr ausgeführt, d.h. nicht aufweitbar, so dass sich durch die Druckschwankungen im Leitungssystem nur die Testabschnitte aufweiten, da nur diese bei einer Drucksteigerung nachgeben. Das Fluid in der Leitung ist insbesondere bei Flüssigkeiten blasenfrei, d.h. gasfrei gehalten, da sonst Druckverfälschungen auftreten, wenn sich das in Blasen befindliche Gasvolumen komprimiert und daher die Druckschwankungen sich nicht wunschgemäß möglichst einzig auf den Testabschnitt auswirken.

Durch eine Erhöhung der Drosselung bzw. eine Erhöhung der Flankensteilheit im zeitlichen Verlauf der Drosselung, d.h. eine stärkere bzw. schnellere Drosselung des Volumenstroms, ergibt sich eine verstärkte stromaufwärtige Druckerhöhung. Durch eine Veränderung der genannten Parameter der Drosselung können daher verschieden starke Druckschwankungen und Druckverläufe eingestellt werden. Zur verschieden starken Drosselung können beispielsweise Absperrventile oder sonstige Drosselarmaturen verwendet werden, die einen verfügbaren Strömungsquerschnitt für das Fluid verringern.

In einer bevorzugten Ausführungsform tritt das Fluid als Fluidstrahl entlang einer Fluidbahn bzw. eines Fluidweges aus einer dem Auslass stromabwärts nachfolgenden Auslassöffnung in einen Freiraum aus. Zur verschieden starken Drosselung wird im Freiraum ein Prallkörper in veränderlichen Relativpositionen zur Fluidbahn in diesen und damit in den Fluidstrahl eingebracht. So trifft zumindest zeitweise zumindest ein Teil des Fluidstrahls auf den Prallkörper auf. Der Auftreffort des Fluidstrahls auf den Prallkörper, also der Teil des Prallkörpers, der von Fluid in Form des Fluidstrahls getroffen wird, bildet dann eine Prallfläche aus. Mit anderen Worten ist der Prallkörper zumindest zeitweise von zumindest einem Teil des aus dem Auslass austretenden Fluids im Fluidstrahl angeströmt. Durch das Auftreffen auf dem Prallkörper erfährt der Fluidstrahl einen Rückstau, was zu dem o.g. Druckstoß führt. Das "veränderliche" Einbringen des Prallkörpers schließt jedwede Veränderlichkeit ein, z.B. eine veränderte Raumlage bzw. Orientierung des Prallkörpers in der Fluidbahn. Eingeschlossen ist auch ein zeitweises vollständiges Entfernen des Prallkörpers aus dem Fluidstrahl, so dass der Fluidstrahl zeitweise ungehindert aus dem Auslass austreten kann und sich kein Rückstau ergibt. Denkbar ist beispielsweise eine lineare Hin- und Herbewegung des Prallkörpers, wobei die Bewegungslinie hierbei in beliebiger Orientierung zur Strahlachse des Fluidstrahls liegen kann, so lange ein Druckstoßeffekt entsteht. Das veränderliche Einbringen des Prallkörpers in die Fluidbahn ist besonders einfach und verschleißfrei durchführbar.

Das veränderliche Einbringen ist außerdem im Sinne einer Veränderung der relativen Lage von Prallkörper und Auslassöffnung zueinander zu verstehen. In einem festen Bezugssystem wird also die Prallfläche und/oder der Fluidstrahl bzw. die Fluidbahn bewegt, um verschiedene Relativpositionen zwischen Prallkörper und Fluistrahl zu erzeugen. Die Veränderung der Fluidbahn erfolgt insbesondere durch Veränderung der die Fluidbahn durch Lenkung des Fluidstrahls bestimmenden Auslassöffnung. Der Prallkörper kann eine Oberfläche beliebiger Form aufweisen. Unter einem Fluidstrahl ist das druckbehaftete Ausströmen des Fluids bei Austritt aus der Auslassöffnung zu verstehen, insbesondere also nicht ein drucklos alleine z.B. durch Schwerkraftwirkung abfließendes Fluid. Entscheidend ist, dass das Einbringen des Prallkörpers in den Fluidstrahl tatsächlich einen Druckstoßeffekt stromaufwärts im Fluid bzw. in der Leitung verursacht.

In einer bevorzugen Variante dieser Ausführungsform wird zur verschieden starken Drosselung - in Strahlrichtung des austretenden Fluidstrahls gesehen - ein Abstand und/oder ein Neigungswinkel der Prallfläche zur Auslassöffnung und/oder ein Überdeckungsgrad zwischen der Querschnittsfläche des Fluidstrahls und der Projektionsfläche der Prallfläche auf die Querschnittsfläche dynamisch verändert. Auch hier ist die "Veränderung" wieder relativ zu verstehen, es wird also in einem festen Bezugssystem der Fluidstrahl bezüglich der Prallfläche und/oder die Prallfläche bezüglich des Fluidstrahles verändert. Abstand, Neigungswinkel und Überdeckungsgrad lassen sich ebenfalls durch sehr einfache und verschleißarme Bewegungen von Prallkörper und/oder Fluidbahn realisieren.

In einer weiteren bevorzugten Variante der oben genannten Ausführungsform werden zur verschieden starken Drosselung der Prallkörper und die Fluidbahn derart relativ zueinander bewegt, dass durch die Bewegung zwischen verschiedenen Relativpositionen jeweils unterschiedliche Teile der Oberfläche des Prallkörpers jeweilige veränderlich in den Fluidstrahl eingebrachte Prallflächen bilden. Mit anderen Worten trifft durch die Relativbewegung von Prallkörper und Fluidstrahl zumindest ein Teil des Fluidstrahls an veränderlichen Abschnitten von dessen Oberfläche, d.h. Prallflächen, auf dem Prallkörper auf. Dies ermöglicht z.B. eine dynamische Bewegung des Prallkörpers in Form eines Hindurchführens durch den Fluidstrahl. Der Prallkörper kann dann insbesondere mit einem Oberflächenprofil bzw. Höhenprofil ausgestattet werden, d.h. dass andere Auftrefforte des Fluids auf dem Prallkörper andere Prallflächen ausbilden. Beispielsweise kann der Prallkörper als rotierende Lochscheibe ausgeführt werden, wobei der Fluidstrahl abwechselnd auf gegenständliche Bereiche des Prallkörpers auftrifft und/oder durch dessen Löcher hindurch tritt. Die Veränderung der Drosselung kann damit besonders einfach durch Strukturierung des Prallkörpers in Kombination mit dessen Bewegung erfolgen.

In einer Variante der genannten Ausführungsform erfolgt die Relativbewegung zwischen Prallkörper und Fluidstrahl durch eine Rotation um eine Rotationsachse, wobei insbesondere der Prallkörper rotiert wird. Die Rotation kann hierbei auch Teil einer Gesamtbewegung sein, also mit anderen, beispielsweise linearen Bewegungen, kombiniert werden. Insbesondere eine Rotationsbewegung des Prallkörpers kann besonders verschleißarm und einfach durch dessen Drehlagerung realisiert werden.

In einer weiteren Variante dieser Ausführungsform kann als Relativbewegung eine Translationsbewegung zwischen Fluidbahn und Prallkörper erfolgen. Auch diese kann Teil einer Gesamtbewegung sein, wie zuvor erläutert. Auch Translationsbewegungen sind verschleißarm möglich. Durch die Veränderung der translatorischen Lage kann insbesondere die Drosselung derart eingestellt werden, dass z.B. ein unteres und oberes Druckniveau für Druckschwankungen festgelegt wird. Die zyklische Druckschwankung zwischen den beiden Niveaus wird dann durch eine Rotationsbewegung erreicht bzw. überlagert.

Zweckmäßig ist der Einsatz eines Druckmodulators gemäß Patentanspruch 8. Der Druckmodulator enthält eine Auslassöffnung, die derart an eine von Fluid durchströmbare Leitung anschließbar ist, dass die Auslassöffnung das Austrittsende der Leitung bildet, durch welche das - vorzugsweise gesamte - die Leitung durchströmende Fluid austritt. Die Auslassöffnung mündet in einen Freiraum. Im Betrieb, d.h. wenn Fluid die Leitung durchströmt, tritt Fluid aus dem Auslass in den Freiraum aus und bildet im Freiraum einen Fluidstrahl, der sich entlang einer Fluidbahn durch den Freiraum bewegt. Mit anderen Worten erfüllt das Fluid nach dem Austritt aus der Auslassöffnung die Fluidbahn in Form des Fluidstrahls. Der Druckmodulator weist außerdem einen zeitlich dynamisch veränderlich in die Fluidbahn einbringbaren Prallkörper auf. Zumindest zeitweise, d.h. wenn zumindest ein Teil des Prallkörpers in die Fluidbahn eintaucht, bildet der Prallkörper eine Prallfläche aus, auf die im Betrieb zumindest ein Teil des Fluidstrahls auftrifft. Mit anderen Worten ist die Prallfläche derjenige Teil des Prallkörpers, der von Fluid des Fluidstrahls angeströmt wird.

Durch das Auftreffen auf die Prallfläche wird der Volumenstrom des Fluids zeitlich dynamisch verschieden stark gedrosselt. So entstehen stromaufwärts, z.B in einer Leitung, an die der Druckmodulator angeschlossen ist, zeitlich dynamische Druckschwankungen im Fluid. Der erfindungsgemäße Druckmodulator eignet sich daher besonders zum Anschluss an den Auslass einer Leitung bzw. eines Leitungssystems, um die zeitlich dynamisch verschieden starke Drosselung des Fluids im oben beschriebenen Verfahren zu bewirken. Der Druckmodulator kann insbesondere so ausgeführt werden, dass dessen einzig bewegliches Teil der Prallkörper ist. Hierbei kann, wie oben bereits ausgeführt, der Prallkörper besonders verschleißarm bewegt werden, so dass sich der Druckmodulator insbesondere für den oben erwähnten Stent-Test eignet, der viele Millionen Druckschwankungen benötigt. Ansonsten gelten für den Druckmodulator bzw. dessen Betrieb sinngemäß die Ausführungen zum erfindungsgemäßen Verfahren.

Bei einer bevorzugten Ausführungsform ist der Abstand und/oder der Neigungswinkel der Prallfläche zum Auslass und/oder der Überdeckungsgrad zwischen der Querschnittsfläche der Fluidbahn und der Projektionsfläche der Prallfläche auf diese Querschnittsfläche veränderbar.

In einer weiteren bevorzugten Ausführungsform sind Prallkörper und Fluidbahn, insbesondere die die Fluidbahn bestimmende Auslassöffnung, derart relativ zueinander verstellbar bzw. bewegbar, dass durch die Verstellung bzw. Bewegung zwischen verschiedenen Relativpositionen jeweils unterschiedliche Teile der Oberfläche des Prallkörpers jeweilige veränderlich in die Fluidbahn ragende Prallflächen bilden.

In einer Variante dieser Ausführungsform sind Prallkörper und Fluidbahn, insbesondere die die Fluidbahn bestimmende Auslassöffnung, relativ zueinander um eine Rotationsachse rotierbar. Rotationsbewegungen können besonders verschleißarm und konstruktiv einfach ausgeführt werden, so dass auf besonders günstige Weise jeweils unterschiedliche Teile der Oberfläche des Prallkörpers veränderlich in die Fluidbahn als Prallflächen einbringbar sind. Als besonders einfache Variante erscheint hierbei, lediglich den Prallkörper zu rotieren.

In einer weiteren Variante dieser Ausführungsform ist alternativ oder zusätzlich die die Fluidbahn bestimmende Auslassöffnung exzentrisch um eine insbesondere parallel zu einer Strahlrichtung der Fluidbahn verlaufende Rotationsachse rotierbar. Exzentrische Bewegungen lassen sich mechanisch einfach ausführen und hinsichtlich einer Fluiddichtheit einfach realisieren. Durch die Bewegung der Auslassöffnung verändert sich auch die Fluidbahn und somit insbesondere bei ortsinvariantem Prallkörper auch der Auftreffort des Fluidstrahls auf den Prallkörper, also die Prallfläche. Durch Gestaltung des Prallkörpers mit diesbezüglich unterschiedlichen Prallflächen können so wiederum Parameter der Druckschwankungen verändert werden. Die Ortsinvarianz des Prallkörpers schließt dabei z.B. eine In-Sich-Bewegung des Prallkörpers ein, z.B. die Rotation eines im Umriss rotationssymmetrischen Prallkörpers wie einer Lochscheibe.

In einer weiteren bevorzugten Ausführungsform sind Prallkörper und Fluidbahn, insbesondere die die Fluidbahn bestimmende Auslassöffnung, translatorisch zueinander verschiebbar.

In einer weiteren Ausführungsform weist der Prallkörper eine Oberflächentopographie, insbesondere ein Höhenprofil und/oder Durchbrechungen auf, die derart über den Prallkörper verteilt ist, dass bei dessen veränderlicher Einbringung in die Fluidbahn verschiedene Abschnitte der Oberflächentopographie abwechselnd in der Fluidbahn liegen. Ein Höhenprofil ist hier bezogen auf den Abstand der Oberfläche des Prallkörpers zur Auslassöffnung zu verstehen. Die Oberflächentopographie erstreckt sich beispielsweise entlang einer Kurve auf der Oberfläche des Prallkörpers, wobei auf der Kurve verschiedene Prallflächen ausgebildet werden, auf denen im Betrieb der Fluidstrahl auftrifft.

Mit anderen Worten ist der Prallkörper also profiliert bzw. geformt, indem beispielsweise Vertiefungen, Erhebungen oder Durchbrechungen in den Prallkörper eingebracht sind. Z.B. ist der Prallkörper eine Scheibe mit Vertiefungen, insbesondere Durchtrittsöffnungen, oder einer Profilkurve, d.h. einem Höhenprofil, welches in Umfangsrichtung um eine Rotationsachse ausgebildet ist. Diese Ausführungsform ist insbesondere sinnvoll in Kombination mit Ausführungsformen, bei denen der Prallkörper um eine Achse parallel zur Strahlachse des Fluidstrahls rotiert wird.

Für die genannten Ausführungsformen des Druckmodulators und deren Vorteile gelten darüber hinaus die obigen Erläuterungen im Zusammenhang mit dem erfindungsgemäßen Verfahren.

Hinsichtlich der Leitungsanordnung wird die Aufgabe gelöst durch eine Leitungsanordnung gemäß Patentanspruch 7 mit einer Leitung, die einen Einlass und einen Auslass aufweist. Die Leitung ist zum Durchströmen mit einem Fluid vom Einlass zum Auslass ausgebildet. Die Leitung ist als Leitungssystem einer Testanordnung für einen medizinischen Stent ausgebildet, wobei das Leitungssystem dann einen Testabschnitt aufweist. Dieser ist durch eine Druckschwankung in Form einer Erhöhung des Drucks des Fluids aufweitbar. Der Stent ist im Testabschnitt platzierbar. Die Leitungsanordnung weist eine Fördereinrichtung für das Fluid auf, um am Einlass das Fluid fördersteif in den Einlass der Leitung zu fördern. Die Leitungsanordnung weist außerdem ein Drosselelement für das Fluid auf, um - insbesondere am bzw. in unmittelbarer Nähe des Auslasses, insbesondere stromabwärts - am Auslass den Volumenstrom des aus dem Auslass strömenden Fluids zeitlich dynamisch, insbesondere periodisch, verschieden stark zu drosseln. Dies dient dazu, und um durch die zeitlich dynamische Änderung der Drosselung stromaufwärtig in der Leitung dynamische Druckschwankungen des Fluids zu erzeugen.

Die Leitungsanordnung eignet sich daher besonders zur Durchführung des erfindungsgemäßen Verfahrens und damit insbesondere als besonders verschleißfreie Testanordnung für medizinische Stents mit den oben erwähnten Vorteilen. Das Drosselelement ist beispielsweise ein Proportionalventil oder ein anderes, den Volumenstrom des Fluids durch Änderung eines Durchtrittsquerschnitts für das Fluid drosselndes Drosselelement. Dieses kann dem Auslass insbesondere in einem anschließenden Leitungsstück stromabwärts nachgeschaltet sein.

In einer bevorzugten Ausführungsform ist das Förderelement eine Fluid in den Eingang speisende, fördersteif ausgelegte Pumpe, insbesondere eine Zahnradpumpe. Im Gegensatz zu einer Membranpumpe, welche bei einem Stent-Dauertest Verschleiß an der Membran unterliegen würde, ist eine Zahnradpumpe besonders verschleißarm.

In einer weiteren bevorzugten Ausführungsform ist das Drosselelement ein dem Auslass der Leitung stromabwärts nachgeschalteter Druckmodulator, wie er oben beschrieben wurde, welcher die entsprechend erwähnten Vorteile mit sich bringt.

Für eine weitere Beschreibung der Erfindung wird auf die Ausführungsbeispiele der Zeichnung verwiesen. Es zeigen, jeweils in schematischer Prinzipdarstellung:
Fig. 1 eine Leitungsanordnung bzw. Testanordnung für einen Stent
Fig. 2 einen Druckmodulator
Fig. 3 ein Detail des Druckmodulators aus Fig. 2 im Querschnitt
Fig. 4 einen alternativen Druckmodulator mit Lochscheibe
Fig. 5 einen weiteren alternativen Druckmodulator mit exzentrischer Verstellung der Auslassöffnung
Fig. 6 eine alternative Testanordnung für Stents
Fig. 7 eine weitere alternative Testanordnung für Stents

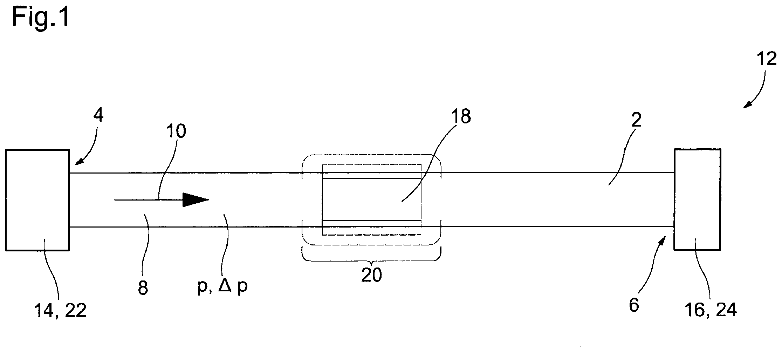

Figur 1 zeigt eine Leitung 2 mit einem Einlass 4 und einem Auslass 6. Durch die Leitung 2 strömt ein Fluid 8 vom Einlass 4 zum Auslass 6 in einer Strömungsrichtung in Richtung des Pfeils 10. Eine Leitungsanordnung 12 enthält neben der Leitung 2 eine Fördereinrichtung 14 für das Fluid 8, um dieses fördersteif in den Einlass 4 der Leitung 2 zu fördern. Weiterhin enthält die Leitungsanordnung 12 ein Drosselelement 16 für das Fluid 8. Das Drosselelement 16 ist dem Auslass 6 stromabwärts nachgeschaltet und drosselt den Volumenstrom des gesamten, aus dem Auslass 6 austretenden Fluids 8. Die Drosselung erfolgt zeitlich dynamisch, d.h. im zeitlichen Wechsel verschieden stark, insbesondere periodisch. Durch die Änderung der Stärke der Drosselung entstehen stromaufwärtig vom Drosselelement 16, d.h. entgegen der Richtung des Pfeils 10, in der Leitung 2 zeitlich dynamische Druckschwankungen Δp des Drucks p des Fluids 8 in der Leitung 2.

Die Leitungsanordnung 12 ist in einer alternativen Ausführungsform Teil einer in Fig. 1 nicht weiter dargestellten Testanordnung 17 für einen medizinischen Stent 18. Die Leitung 2 besitzt dann einen Testabschnitt 20, in welchem der Stent 18 platziert, nämlich radial auswärts federnd vorgespannt ist. Der Testabschnitt 20 der Leitung 2 ist durch eine Druckschwankungen Δp in Form einer Erhöhung des Drucks p im Fluid 8 radial aufweitbar, was in Figur 1 gestrichelt angedeutet ist. Der Stent 18 folgt dann der Aufweitung im Testabschnitt 20 und wird dadurch mechanisch verformt. Bei einem Abfall des Drucks p erfolgt eine gegensätzliche Bewegung. Durch zyklische Druckschwankungen Δp wird der Stent 18 daher einem Ermüdungstest unterzogen.

Die Fördereinrichtung 14 ist im Beispiel eine, das Fluid 8 in den Einlass 4 speisende Pumpe 22, die fördersteif ausgelegt ist, insbesondere eine Zahnradpumpe. Das Drosselelement 16 ist im Beispiel ein an den Auslass 6 angeschlossener Druckmodulator 24.

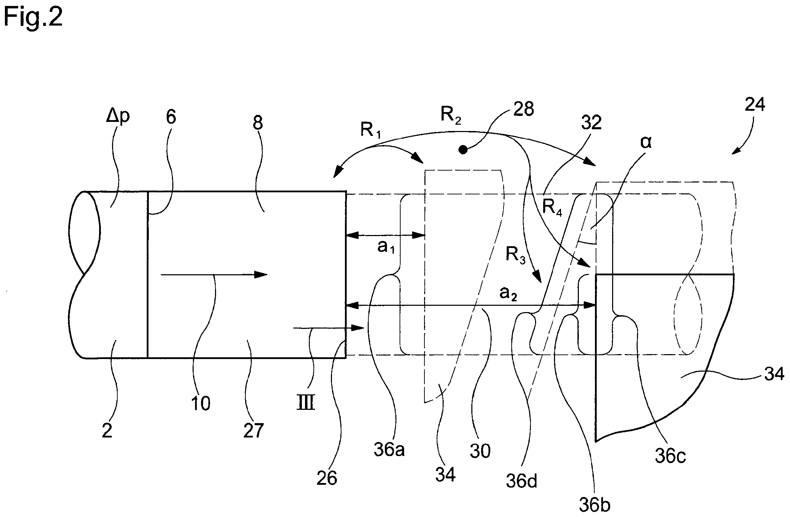

Fig. 2 zeigt den Druckmodulator 24 aus Fig. 1 näher im Detail. Der Druckmodulator enthält eine Auslassöffnung 26, die, im Beispiel als offenes Ende eines Rohrstückes 27 gebildet ist, das an die mit Fluid 8 durchströmte Leitung 2 bzw. deren Auslass 6 angeschlossen ist. Sämtliches, durch den Auslass 6 strömendes Fluid 8 tritt damit aus der Auslassöffnung 26 aus. Die Auslassöffnung 26 mündet in einen Freiraum 28 des Druckmodulators 24. Im Betrieb, d.h. wenn Fluid 8 durch die Leitung 2 strömt, tritt das Fluid an der Auslassöffnung 26 in den Freiraum 28 aus und bildet dabei einen Fluidstrahl 30, der sich entlang einer dreidimensionalen, räumlich ausgedehnten Fluidbahn 32 durch den Freiraum 28 bewegt; in Fig. 2 ist die Fluidbahn 32 gestrichelt angedeutet. Ein Prallkörper 34 des Druckmodulators 24 ist in zeitlich veränderlicher Weise in die Fluidbahn 32 einbringbar bzw. auch aus diesem entfernbar. Im Betrieb, d.h. bei Vorhandensein des Fluidstrahls 30 trifft immer dann, wenn zumindest ein Teil des Prallkörpers 34 in die Fluidbahn 32 ragt, zumindest ein Teil des Fluidstrahls 30, d.h. des Fluids 8 auf den Prallkörper 34 auf. Der jeweilige Oberflächenbereich des Prallkörpers 34, der jeweils aktuell vom Fluidstrahl 30 getroffen wird, bildet dann eine jeweilige Prallfläche 36a-c des Prallkörpers 34 aus.

Fig. 2 zeigt vier verschiedene Relativpositionen R₁₋₄ zwischen Prallkörper 34 (eine ausgezogen und zwei gestrichelt) und Fluidbahn32 bzw. Fluidstrahl 30. Dabei bilden sich am Prallkörper 34 jeweils unterschiedliche Prallflächen 36a-d aus. Durch das zeitlich dynamisch veränderliche Einbringen des Prallkörpers 34 in die Fluidbahn 32 in bzw. zwischen den gezeigten Relativpositionen R₁₋₃ entstehen im Betrieb Druckstöße des Drucks p im Fluid 8, welche sich stromaufwärts, d.h. entgegen der Strömungsrichtung des Fluids 8, also entgegen der Richtung des Pfeils 10 in Form von Druckschwankungen Δp in die Leitung 2 fortsetzen.

Durch die translatorische und/oder rotatorische Bewegung und/oder Neigungsbewegung des Prallkörpers 34 ändert sich in einer ersten Variante der - in Strahlrichtung des Fluidstrahls 30 bzw. entlang der Fluidbahn 32 gesehene - Abstand a_{1,2} zwischen Prallfläche 36a-d und Auslassöffnung 26. Alternativ oder zusätzlich ändert sich der Neigungswinkel α zwischen den Prallfläche 36a-d und der Auslassöffnung 26.

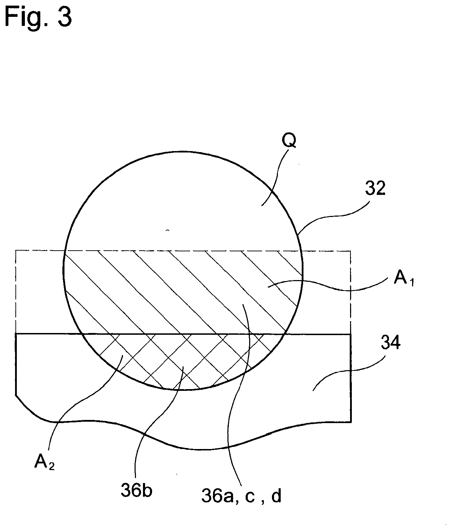

Fig. 3 zeigt bei Blickrichtung entlang des Pfeils III in Fig. 2, also in Strahlrichtung, wie sich durch verändertes Einbringen des Prallkörpers 34 in verschiedenen Relativpositionen R₁₋₃ alternativ oder zusätzlich auch ein Überdeckungsgrad G=Q/A_{1,2} verändert. Dieser beschreibt das Flächenverhältnis zwischen der Querschnittsfläche Q der Fluidbahn 32 und der Projektionsflächen A_{1,2} der Projektionen der Prallflächen 36a-d auf diese Querschnittsfläche Q bzw. die entsprechende Querschnittsebene. In Fig. 3 sind die unterschiedlichen Positionen des Prallkörpers 34 ausgezogen und durch Strichelung dargestellt, die sich ergebenden unterschiedlichen Prallflächen 36a-d bzw. deren Projektionsflächen A_{1,2} durch unterschiedliche Schraffuren.

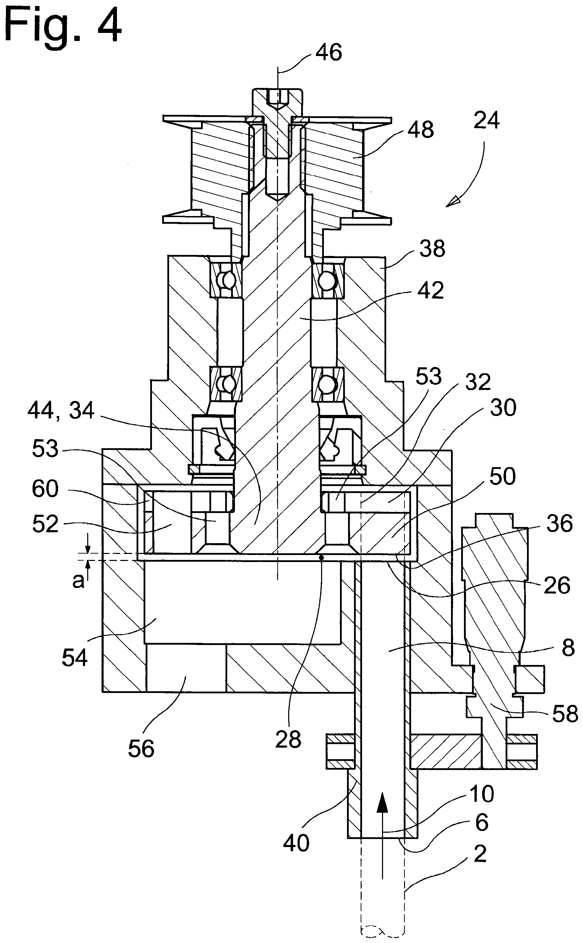

Fig. 4 zeigt eine alternative Ausführungsform eines Druckmodulators 24 mit einem Gehäuse 38, in dem ein Staurohr 40 vorgesehen ist. Das Staurohr 40 ist funktional dem zusätzlichen Rohrstück 27 aus Fig. 2 ähnlich, ist am Auslass 6 der Leitung 2 angeschlossen und endet seinerseits in der Auslassöffnung 26. Das Gehäuse 38 umschließt den Freiraum 28, in welchem der Prallkörpers 34 angeordnet ist. Die Fluidbahn 32 erstreckt sich beginnend an der Auslassöffnung 26 in den Freiraum 28. Der Prallkörper 34 ist hier als Kreisscheibe 44 realisiert, welche einstückig mit einer Welle 42 verbunden ist. Welle 42 und Scheibe 44 sind um eine Rotationsachse 46 rotierbar. Ein Rotationsantrieb erfolgt in nicht näher erläuterter Weise über einen nicht dargestellten Riemen und eine Riemenscheibe 48 oder alternativ durch andere nicht dargestellte Antriebsvarianten, z.B. einen Direktantrieb. Die Scheibe 44 besitzt über ihren Umfang verteilt massive Abschnitte 50 und Durchbrechungen 52 in Form von Durchgangsbohrungen. Durch die Rotation der Scheibe 44 um die Rotationsachse 46 gelangen abwechselnd die massiven Abschnitte 50 oder die Durchbrechungen 52 in den Bereich der Fluidbahn 32. Liegt ein massiver Abschnitt 50 in der Fluidbahn 32, bildet sich an diesem eine Prallfläche 36 am Prallkörper 34 aus, wie in Figuren 2 und 3 für die Prallflächen 36a-c erläutert. Liegt eine Durchbrechung 52 im Bereich der Fluidbahn 32, erfährt der Fluidstrahl 30 keinen nennenswerten Widerstand und kann ungehindert durch die Durchbrechung 52 hindurch treten, ohne auf den Prallkörper 34 aufzutreffen. Eine Prallfläche 36 ist dann nicht vorhanden.

Der Prallkörper 34, d.h. dessen massiver bzw. körperlicher. Bereich, wird also bei dessen Rotation zeitlich dynamisch abwechselnd in die Fluidbahn 32 eingebracht oder nicht, um dadurch zeitlich dynamisch abwechselnd Prallflächen 36 auszubilden oder nicht. Hierdurch entstehen zeitlich abwechselnd Druckstöße im Fluid 8, die sich entgegen der Richtung des Pfeils 10 in die Leitung 2 fortsetzen und dort Druckschwankungen Δp zur Folge haben. Das aus der Auslassöffnung 26 ausgetretene Fluid 8 sammelt sich nach dem Auftreffen oder dem Durchtritt durch den Prallkörper 34 drucklos in einer Sammelkammer 54, aus der es durch einen Ablauf 56 drucklos abfließen kann und so beispielsweise in einem Kreislauf wieder zur Pumpe 22 gelangt, von der es wieder in die Leitung 2 gefördert wird.

Der Abstand a in Form eines Spaltmaßes zwischen dem rotierenden Prallkörper 34 und der Auslassöffnung 26 kann verändert werden, indem das Staurohr 40 mit Hilfe eines Motorantriebs 58 in oder entgegen der Richtung des Pfeils 10 von der Scheibe 44 weg oder auf diese zu bewegt wird. Der Abstand a bestimmt hierbei das obere Druckniveau der sich ergebenden Druckschwankungen Δp. Ein entsprechendes unteres Druckniveau ist dadurch verstellbar, dass mit Hilfe einer um die Rotationsachse 46 relativ zur Scheibe 44 rotierbaren Blendenscheibe 60 der effektive Querschnitt der Durchbrechungen 52 veränderbar ist. Mit anderen Worten werden zwei Lochscheiben gegeneinander um die Achse 46 verdreht, um den jeweiligen effektiven Lochquerschnitt zu kleinern oder zu vergrößern. Eine derartige Verstellung erfolgt z.B. nur einmalig oder zwischenzeitlich bei der Justierung oder Wartung der Anlage, jedoch nicht während des regulären Betriebs. Die Verdrehung beider Scheiben relativ zueinander erfordert den Stillstand der Anlage, da die Scheiben durch eine in Fig. 4 lediglich angedeutete, nicht näher erläuterte Verschraubung 53 gegeneinander drehfixiert sind, die zur Veränderung der Drehlage beider Scheiben gelöst und wieder befestigt werden muss. Entspricht bei einer derartigen Lochscheibe in Umfangsrichtung gesehen der Abstand zwischen den Löchern etwa dem Lochdurchmesser, so ergibt sich als Druckverlauf eine Sinuskurve.

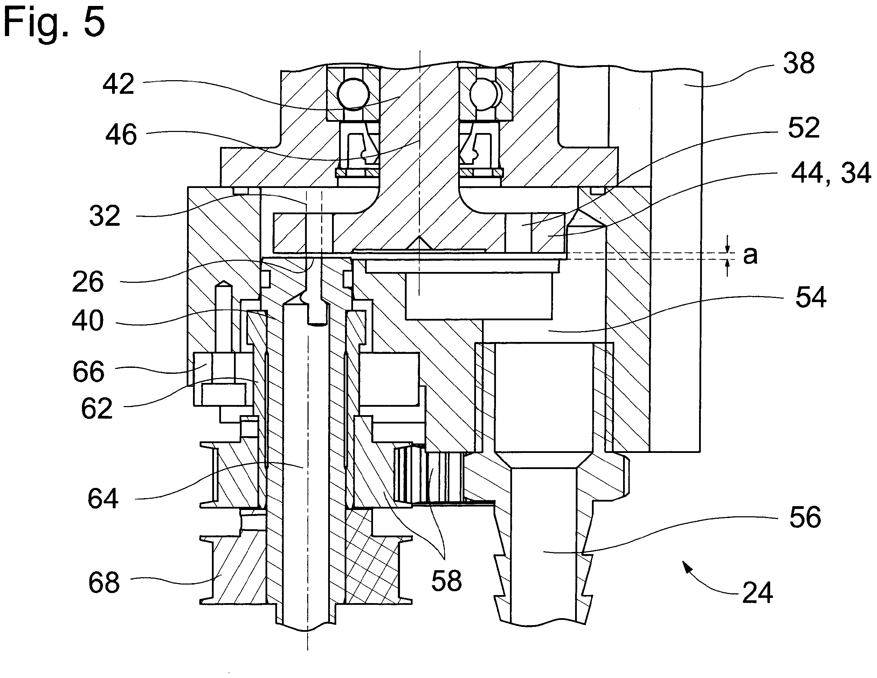

Fig. 5 zeigt einen Ausschnitt einer weiteren alternativen Ausführungsform eines Druckmodulators 24, welcher hinsichtlich Aufbau und Funktionsprinzip der Ausführungsform gemäß Fig. 4 dahingehend ähnelt, dass eine rotierende Scheibe 44 mit Durchbrechungen 52 und massiven, festen Abschnitten 50 (vgl. Fig. 4, in Fig. 5 nicht sichtbar) als Prallkörper 34 dient und um eine Rotationsachse 46 derart rotiert wird, dass die Durchbrechungen 52 und massiven Abschnitte 50 der Scheibe 44 abwechselnd der Auslassöffnung 26 des Staurohres 40 gegenüberliegen. Die Einstellung des Abstandes a erfolgt in der o.g. Weise durch einen Motorantrieb 58, der hier die Rotation eines Führungskörpers 62 um eine Rotationsachse 64 bewirkt. Aufgrund eines Gewindeeingriffs zwischen dem Führungskörper 62 und einem fest am Gehäuse 38 angebrachten Gegenkörpers 66 erfolgt eine Verschiebung des Führungskörpers 62 in axialer Richtung der Rotationsachse 64 unter Mitnahme des Staurohres 40. Diese Einstellmöglichkeit dient wieder zur Einstellung des oberen Druckniveaus der Druckschwankungen Δp.

Das untere Druckniveau wird in der Ausführungsform gemäß Fig. 5 auf alternative Weise eingestellt, nämlich durch Rotation des Staurohres 40 um die Rotationsachse 64 mit Hilfe der Riemenscheibe 68. Um eine Einstellbarkeit zu erreichen, ist die Auslassöffnung 26 am Staurohr 40 exzentrisch bezüglich der Rotationsachse 64 angeordnet. Bei Rotation des Staurohres 40 erfolgt so eine Verstellung der Position der Auslassöffnung 26 zur Scheibe 44, d.h. deren Radialabstand zur Rotationsachse 46 verändert sich. Hierdurch verändert sich auch die Position der Fluidbahn 32 relativ zur Scheibe 44 und damit relativ zu deren Durchbrechungen 52 bzw. massiven Abschnitten 50. Die Überdeckung zwischen dem Querschnitt der Fluidbahn 32 und den Durchbrechungen 52 verändert sich. Hierdurch verändert sich auch die Größe der am Prallkörper 34 entstehenden Prallflächen 36a-c (in Fig. 5 nicht dargestellt), wie gemäß der Figuren 2 und 3 erläutert. Gegenüber der Ausführungsform gemäß Figur 4 hat dies den Vorteil, dass auch das untere Druckniveau im Betrieb des Druckmodulators 24 unterbrechungsfrei durch Betätigung der Riemenscheibe 68 verstellbar ist. Das zylindrisch gestaltete Staurohr 40 mit Exzentereigenschaft ist leicht, z.B. durch herkömmliche O-Ring-Dichtungen gegen Austritt von Fluid 8 abdichtbar.

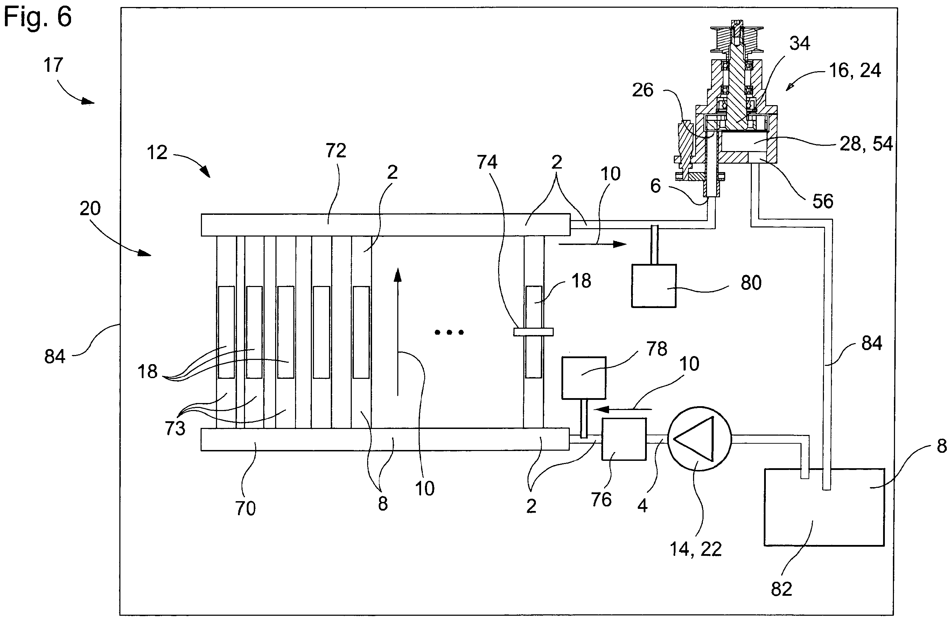

Fig. 6 zeigt eine Leitungsanordnung 12 als Teil einer Testanordnung 17 für Stents 18. Die Leitungsanordnung 12 enthält hier mehrere Leitungen 2 für Fluid 8, die unter anderem als Verteilerschiene 70 und Sammelschiene 72, sowie in Form mehrerer Testabschnitte 20 ausgeführt sind, die jeweils von der Verteilerschiene 70 angeströmt werden und in die Sammelschiene 72 münden. Die Testabschnitte 20 sind im Beispiel künstliche Gefäße 73, z.B. Silikonschläuche, in denen die Stents 18 radial auswärts federnd vorgespannt eingesetzt sind. Mit Ausnahme der künstlichen Gefäße 73 sind sämtliche restlichen Leitungen 2 starr ausgeführt, d.h. dehnen sich bei Druckschwankungen Δp im Fluid 8 nicht aus, sind also formstabil. Nur die künstlichen Gefäße dehnen sich unter Mitnahme der Stents 18 aus, wodurch diese einem Ermüdungstest durch radiale Expansion und Kompression unterzogen werden. Zur Ermittlung der Durchmesser einzelner Stents 18 ist symbolisch ein Messgerät 74 angedeutet. In der Testanordnung 17 ist außerdem ein Volumenstrommessgerät 76, ein Temperaturmessgerät 78 und ein Druckmessgerät 80 - jeweils für das Fluid 8 und symbolisch angedeutet - angeschlossen.

Das Fluid 8 ist lediglich im Bereich stromaufwärts entlang der Pfeile 10 zwischen dem Einlass 4 und dem Auslass 6 bzw. der Auslassöffnung 26 druckbehaftet. Bereits im Freiraum 28 bzw. in der Sammelkammer 54 und in einem Vorratsbehälter 82 sowie in einer Rücklaufleitung 84 ist das Medium 8 drucklos vorhanden. Die Fördereinrichtung 14 entnimmt daher Fluid 8 aus dem Vorratsbehälter 82, um dieses fördersteif in die Leitungsanordnung 12 einzuspeisen. Der Vorratsbehälter 82 sowie eine Kammer 84, die die Testanordnung 17 umgibt, sind beheizbar.

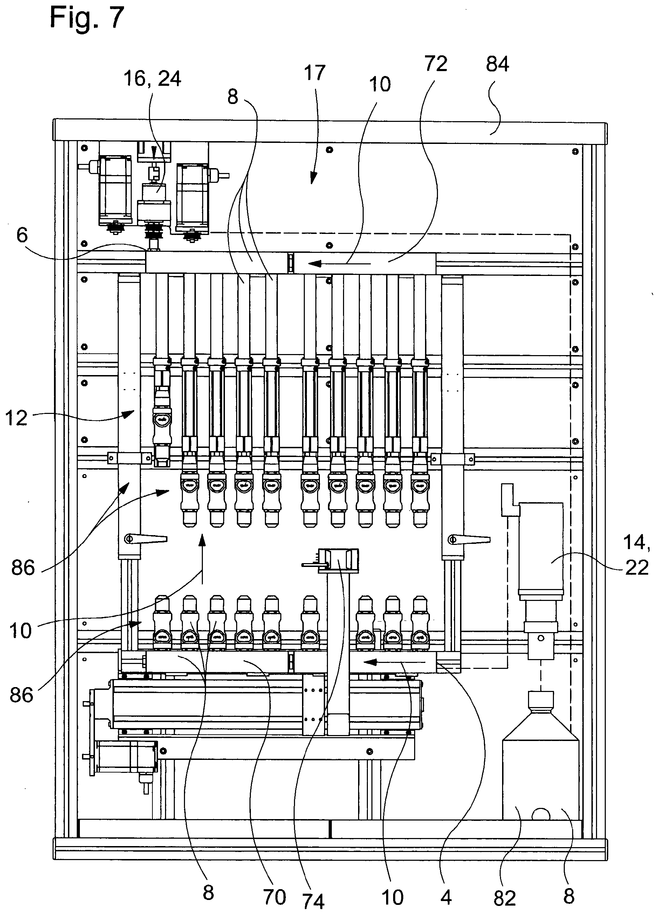

Die alternative Testanordnung 17 gemäß Fig. 7 unterscheidet sich von der Testanordnung 17 aus Fig. 6: Die Verteilerschiene 70 ist auf die Sammelschiene 72 zu oder von dieser weg bewegbar. Außerdem sind an der Verteilerschiene 70 und der Sammelschiene 72 zunächst weitere starre Leitungen 2 angeschlossen, welche in jeweiligen Steckern oder Buchsen von Schnellkupplungen 86 enden. Zwischen die jeweiligen Schnellkupplungen 86 sind dann die nicht dargestellten, flexiblen künstlichen Gefäße 73 mit Stents 18 einsetzbar. Die an der Sammelschiene 72 angeordneten Schnellkupplungen 86 sind außerdem nochmals separat auf die Sammelschiene 72 zu oder von dieser weg bewegbar, um im Betrieb auch einzelne künstliche Gefäße bzw. Stents einsetzen oder herausnehmen zu können. Das Messgerät 74 ist in dieser Ausführungsform verschiebbar angeordnet, um wahlweise verschiedene künstliche Gefäße 73 bzw. Stents 18 vermessen zu können. Die Leitungsverbindungen zwischen Druckmodulator 24, Vorratsbehälter 82, Pumpe 22 und Einlass 4 nur gestrichelt angedeutet.

### Bezugszeichenliste

2 Leitung
4 Einlass
6 Auslass
8 Fluid
10 Pfeil
12 Leitungsanordnung
14 Fördereinrichtung
16 Drosselelement
17 Testanordnung
18 Stent
20 Testabschnitt
22 Pumpe
24 Druckmodulator
26 Auslassöffnung
27 Rohrstück
28 Freiraum
30 Fluidstrahl
32 Fluidbahn
34 Prallkörper
36,36a-d Prallfläche
38 Gehäuse
40 Staurohr
42 Welle
44 Scheibe
46 Rotationsachse
48 Riemenscheibe
50 Abschnitt
52 Durchbrechung
53 Verschraubung
54 Sammelkammer
56 Ablauf
58 Motorantrieb
60 Blendenscheibe
62 Führungskörper
64 Rotationsachse
66 Gegenkörper
68 Riemenscheibe
70 Verteilerschiene
72 Sammelschiene
73 Gefäß
74 Messgerät
76 Volumenstrommessgerät
78 Temperaturmessgerät
80 Druckmessgerät
82 Vorratsbehälter
84 Kammer
86 Schnellkupplung

Δp Druckschwankung
p Druck
Q Querschnittsfläche
a,a_{1,2} Abstand
R₁₋₄ Relativposition
G Überdeckungsgrad
α Neigungswinkel
A₁,₂ Fläche

## Patentansprüche

1. Verfahren zum Erzeugen von Druckschwankungen (Δp) in einem Fluid (8) in einem Leitungssystem (12) einer Testanordnung (17) für einen medizinischen Stent (18) mit einem durch eine Erhöhung des Drucks (p) des Fluids (8) aufweitbaren Testabschnitt (20), in dem der Stent (18) platzierbar oder platziert ist, mit folgendem Schritt:
- die Leitung (2) wird von einem Einlass (4) zu einem Auslass (6) hin vom Fluid (8) durchströmt,
**gekennzeichnet durch die weiteren, folgenden Schritte:**
- am Einlass (4) wird das Fluid (8) fördersteif in den Einlass (4) der Leitung (2) gefördert,
- am Auslass (6) wird der Volumenstrom des aus dem Auslass (6) strömenden Fluids (8) zeitlich dynamisch, insbesondere periodisch, verschieden stark gedrosselt, wobei durch die Änderung der Drosselung stromaufwärtig in der Leitung (2) zeitlich dynamische Druckschwankungen (Δp) im Fluid (8) erzeugt werden.

2. Verfahren nach Anspruch 1, bei dem das Fluid (8) als Fluidstrahl (30) entlang einer Fluidbahn (32) aus einer dem Auslass (6) stromabwärts folgenden Auslassöffnung (26) in einen Freiraum (28) austritt, und zur verschieden starken Drosselung im Freiraum (28) ein Prallkörper (34) in veränderlichen Relativpositionen (R₁₋₄) zur Fluidbahn (32) in diesen eingebracht wird, so dass zumindest ein Teil des Fluidstrahls (30) zumindest zeitweise an einer Prallfläche (36,36a-d) auf den Prallkörper (34) auftrifft.

3. Verfahren nach Anspruch 2, bei dem zur verschieden starken Drosselung ein Abstand (a,a₁,₂) und/oder ein Neigungswinkel (α) der Prallfläche zur Auslassöffnung (26) und/oder ein Überdeckungsgrad (G) zwischen einer Querschnittsfläche (Q) der Fluidbahn (32) und der Projektionsfläche (A₁,₂) der Prallfläche (36,36a-d) auf die Querschnittsfläche (Q) verändert wird.

4. Verfahren nach einem der Ansprüche 2 bis 3, bei dem zur verschieden starken Drosselung der Prallkörper (34) und die Fluidbahn (32) derart relativ zueinander bewegt werden, dass durch die Bewegung zwischen verschiedenen Relativpositionen (R₁₋₄) jeweils unterschiedliche Teile der Oberfläche des Prallkörpers (34) jeweilige veränderlich in den Fluidstrahl (30) eingebrachte Prallflächen (36,36a-d) bilden.

5. Verfahren nach Anspruch 4, bei dem der Prallkörper (34) und die Fluidbahn (32) durch eine Rotationsbewegung um eine Rotationsachse (46) relativ zueinander bewegt werden, wobei insbesondere der Prallkörper (34) rotiert wird.

6. Verfahren nach einem der Ansprüche 4 bis 5, bei dem der Prallkörper (34) und die Fluidbahn (32) durch eine Translationsbewegung relativ zueinander bewegt werden.

7. Leitungsanordnung (12) für eine Testanordnung (17) für einen medizinischen Stent (18) mit
- einer Leitung (2) mit einem Einlass (4) und einem Auslass (6) zum Durchströmen der Leitung (2) mit einem Fluid (8) vom Einlass (4) zum Auslass (6) und
mit einem durch eine Druckschwankung (Δp) in Form einer Erhöhung des Drucks (p) des Fluids (8) aufweitbaren Testabschnitt (20), in
dem der Stent (18) platzierbar ist,
**gekennzeichnet durch**
- eine Fördereinrichtung (14) für das Fluid (8), um am Einlass (4) das Fluid (8) fördersteif in den Einlass (4) der Leitung (2) zu fördern, und
- ein Drosselelement (16) für das Fluid (8), um einen Volumenstrom des aus dem Auslass (6) strömenden Fluids (8) zeitlich dynamisch, insbesondere periodisch, verschieden stark zu drosseln, um durch die Änderung der Drosselung stromaufwärtig in der Leitung (2) dynamische Druckschwankungen (Δp) des Fluids (8) zu erzeugen.

8. Leitungsanordnung (12) nach Anspruch 7, bei dem die Fördereinrichtung (14) eine
Fluid (8) in den Einlass (4) speisende, fördersteif ausgelegte Pumpe (22),
insbesondere eine Zahnradpumpe, ist und/oder das Drosselelement (16) ein dem Auslass (6) stromabwärts nachgeschalteter Druckmodulator (24) mit einer Auslassöffnung (26) ist, die derart an eine von Fluid (8) durchströmbare Leitung (2) anschließbar ist, dass sie deren Austrittssende für das Fluid (8) bildet, wobei die Auslassöffnung (26) in einen Freiraum (28) mündet, so dass das Fluid (8) im Betrieb einen aus der Auslassöffnung (26) in den Freiraum (28) entlang einer Fluidbahn (32) austretenden Fluidstrahl (30) bildet, und mit einem zeitlich dynamisch veränderlich in die Fluidbahn (32) einbringbaren Prallkörper (34), der zumindest zeitweise eine Prallfläche (36,36a-d) ausbildet, auf die im Betrieb zumindest ein Teil des Fluidstrahls (30) auftrifft.

9. Leitungsanordnung (12) nach Anspruch 8, bei dem der Abstand (a,a_{1,2}) und/oder der Neigungswinkel (α) der Prallfläche zur Auslassöffnung (26) und/oder der Überdeckungsgrad (G) zwischen der Querschnittsfläche (Q) der Fluidbahn (32) und der Projektion der Prallfläche (36,36a-d) auf die Querschnittsfläche (Q) veränderbar ist.

10. Leitungsanordnung (12) nach einem der Ansprüche 8 bis 9, bei dem der Prallkörper (34) und die Fluidbahn (32) derart relativ zueinander verstellbar sind, dass durch die Verstellung zwischen verschiedenen Relativpositionen (R₁₋₄) jeweils unterschiedliche Teile der Oberfläche des Prallkörpers (34) jeweilige veränderlich in die Fluidbahn (32) ragende Prallflächen (36,36a-d) bilden.

11. Leitungsanordnung (12) nach Anspruch 10, bei dem Prallkörper (34) und Fluidbahn (32) relativ zueinander um eine Rotationsachse (46) rotierbar sind, insbesondere der Prallkörper (34) rotierbar ist.

12. Leitungsanordnung (12) nach Anspruch 11, bei dem die Fluidbahn (32) exzentrisch um eine, insbesondere parallel zu einer Strahlrichtung der Fluidbahn (32) verlaufende, Rotationsachse (64) relativ zum Prallkörper (34) rotierbar ist.

13. Leitungsanordnung (12) nach einem der Ansprüche 10 bis 12, bei dem Prallkörper (34) und Fluidbahn (32), insbesondere der die Fluidbahn (32) bestimmenden Auslassöffnung (26), relativ zueinander translatorisch verschiebbar sind.

14. Leitungsanordnung (12) nach einem der Ansprüche 8 bis 13, bei dem der Prallkörper (34) eine Oberflächentopographie, insbesondere ein Höhenprofil und/oder Durchbrechungen (52), aufweist, die derart über den Prallkörper (34) verteilt ist, dass bei dessen veränderlicher Einbringung in die Fluidbahn (32) verschiedene Abschnitte der Oberflächentopographie abwechselnd in der Fluidbahn (32) liegen.

## Claims

1. A method for generating pressure fluctuations (Δp) in a fluid (8) in a line system (12) of a test layout (17) for a medical stent (18), having a test section (20) which can be expanded by increasing the pressure (p) of the fluid (8), in which section the stent (18) can be or is positioned, the method comprising the step:
- the fluid (8) flows through the line (2) from an inlet (4) to an outlet (6),
**characterised by the additional, following steps:**
- at the inlet (4) the fluid (8) is delivered at a constant rate into the inlet (4) of the line (2),
- at the outlet (6), the volumetric flow of the fluid (8) flowing out of the outlet (6) is throttled by varying degrees, dynamically over time, in particular periodically, by changing the throttling upstream in the line (2), temporally dynamic pressure fluctuations (Δp) being generated in the fluid (8).

2. The method according to Claim 1, wherein the fluid (8) emerges as a fluid jet (30) along a fluid path (32) from an outlet opening (26) downstream from the outlet (6) into a clearance (28), and for the throttling of different degrees, an impact body (34) is introduced in the clearance (28) in different positions (R₁₋₄) relative to the fluid path (32) so that at least a portion of the fluid jet (30) impinges on an impact surface (36, 36a-d) of the impact body (34) at least some of the time.

3. The method according to Claim 2, wherein, for the throttling of different degrees, a spacing (a,a_{1,2}) and/or an angle of inclination (α) of the impact surface to the outlet opening (26) and/or a degree of overlap (G) between a cross-sectional area (Q) of the fluid path (32) and the projection surface (A_{1,2}) of the impact surface (36, 36a-d) on the cross-sectional area (Q) is varied.

4. The method according to either of Claims 2 to 3, wherein, for the throttling to different degrees, the impact body (34) and the fluid path (32) are moved relatively to each other such that, due to the movement between different relative positions (R₁₋₄), respectively different parts of the surface of the impact body (34) form impact surfaces (36, 36a-d) respectively introduced variously into the fluid jet (30)

5. The method according to Claim 4, wherein the impact body (34) and the fluid path (32) are moved relative to each other by a rotational movement about an axis of rotation (46), the impact body (34) in particular being rotated.

6. The method according to either of Claims 4 to 5, wherein the impact body (34) and the fluid path (32) are moved relative to one another by a translational movement.

7. A line arrangement (12) for a test layout (17) for a medical stent (18), comprising
- a line (2) having an inlet (4) and an outlet (6) for the flow of a fluid (8) through the line (2) from the inlet (4) to the outlet (6), and having a test section (20) which can be expanded by a pressure fluctuation (Δp) in the form of an increase in the pressure (p) of the fluid (8) and in which the stent (18) can be positioned,
**characterised by**
- a delivery device (14) for the fluid (8) in order to deliver the fluid (8) at the inlet (4) at a constant rate into the inlet (4) of the line (2), and
- a throttle element (16) for the fluid (8) in order to throttle a volumetric flow of the fluid (8) flowing out of the outlet (6) to different degrees dynamically over time, especially periodically, in order to generate dynamic pressure fluctuations (Δp) of the fluid (8) upstream in the line (2) by the change in the throttling.

8. The line arrangement (12) according to Claim 7, wherein the delivery device (14) is a pump (22), in particular a geared pump, designed to feed fluid (8) into the inlet (4) at constant rate, and/or the throttle element (16) is a pressure modulator (24) connected downstream of the outlet (6), having an outlet opening (26) which can be connected to a line (2) through which fluid (8) can flow such that said outlet opening forms its exit end for the fluid (8), the outlet opening (26) discharging into a clearance (28) so that during operation the fluid (8) forms a fluid jet (30) passing out of the outlet opening (26) into the clearance (28) along a fluid path (32), and having an impact body (34) that can be introduced into the fluid path (32) variously, dynamically over time, which impact body forms at least some of the time an impact surface (36, 36a-d) on which at least a portion of the fluid jet (30) impinges during operation.

9. The line arrangement (12) according to Claim 8, wherein the spacing (a, a_{1,2}) and/or the angle of inclination (α) of the impact surface to the outlet opening (26) and/or the degree of overlap (G) between the cross-sectional area (Q) of the fluid path (32) and the projection of the impact surface (36, 36a-d) on the cross-sectional area (Q) is variable.

10. The line arrangement (12) according to either of Claims 8 to 9, wherein the impact body (34) and the fluid path (32) can be adjusted relative to each other so that, by means of the adjustment between different relative positions (R₁₋₄), respectively different parts of the surface of the impact body (34) form impact surfaces (36, 36a-d) respectively protruding variously into the fluid path (32).

11. The line arrangement (12) according to Claim 10, wherein the impact body (34) and the fluid path (32) are rotatable relative to one another about an axis of rotation (46), in particular the impact body (34) is rotatable.

12. The line arrangement (12) according to Claim 11, wherein the fluid path (32) can be rotated eccentrically about an axis of rotation (64), in particular running parallel to a jet direction of the fluid path (32), relative to the impact body (34).

13. The line arrangement (12) according to any of Claims 10 to 12, wherein the impact body (34) and the fluid path (32), in particular the outlet opening (26) determining the fluid path (26), can be moved in translation relative to one another.

14. The line arrangement (12) according to any of Claims 8 to 13, wherein the impact body (34) has a surface topography, in particular a height profile and/or interruptions (52), which is distributed over the impact body (34) such that, when the impact body (34) is introduced variously into the fluid path (32), different sections of the surface topography lie alternately in the fluid path (32).

## Revendications

1. Procédé pour générer des fluctuations de pression (Δp) dans un fluide (8) dans un système de conduite (12) d'un dispositif de test (17) pour un stent médical (18) avec un tronçon de test (20) qui peut être dilaté par une augmentation de la pression (p) du fluide (8), dans lequel le stent (18) est placé ou peut être placé, comprenant les étapes suivantes :
- la conduite est traversée par le fluide (8) depuis une entrée (4) jusqu'à une sortie (6),
**caractérisé par les étapes supplémentaires suivantes :**
- à l'entrée (4) le fluide (8) est introduit selon un flux à volume constant dans l'entrée (4) de la conduite (2),
- à la sortie (6) le flux volumique du fluide (8) s'écoulant en provenance de la sortie (6) est étranglé à des degrés variables, de façon dynamique en fonction du temps, spécialement de façon périodique, dans lequel, au moyen de la modification de l'étranglement, des fluctuations de pression (Δp) dynamiques en fonction du temps sont générées dans le fluide (8) en amont dans la conduite (2).

2. Procédé selon la revendication 1, dans lequel le fluide (8) sort sous forme d'un jet de fluide (30) le long d'un chemin de fluide (32) depuis un orifice de sortie (26) en aval de la sortie (6) dans un espace libre (28), et pour l'étranglement à des degrés différents, un corps d'impact (34) est introduit dans l'espace libre (28) dans des positions relatives variables (R₁₋₄) par rapport au chemin de fluide (32) dans celui-ci, de sorte que au moins une portion du jet de fluide (30) heurte une surface d'impact (36,36a-d) sur le corps d'impact (34) au moins une partie du temps.

3. Procédé selon la revendication 2, dans lequel, pour l'étranglement à des degrés différents, on fait varier un espacement (a,a_{1,2}) et/ou un angle d'inclinaison (α) de la surface d'impact par rapport à l'orifice de sortie (26) et/ou un degré de recouvrement (G) entre une zone de section transversale (Q) du chemin de fluide (32) et la surface de projection (A_{1,2}) de la surface d'impact (36,36a-d) sur la zone de section transversale (Q).

4. Procédé selon l'une des revendications 2 à 3, dans lequel, pour l'étranglement à des degrés différents, le corps d'impact (34) et le chemin de fluide (32) sont déplacés relativement l'un par rapport à l'autre de telle sorte que, chaque fois qu'ils sont déplacés l'un par rapport à l'autre, des parties différentes de la surface du corps d'impact (34) forment des surfaces d'impact (36,36a-d) introduites de façon variable dans le jet de fluide (30) en fonction du mouvement entre les positions relatives différentes (R₁₋₄).

5. Procédé selon la revendication 4, dans lequel le corps d'impact (34) et le chemin de fluide (32) sont déplacés l'un par rapport à l'autre par un mouvement de rotation autour d'un axe de rotation (46), dans lequel le corps d'impact (34) en particulier est mis en rotation.

6. Procédé selon l'une des revendications 4 à 5, dans lequel le corps d'impact (34) et le chemin de fluide (32) sont déplacés l'un par rapport à l'autre par un mouvement de translation.

7. Agencement de conduite (12) pour un dispositif de test (17) pour un stent médical (18), comprenant
- une conduite (2) ayant une entrée (4) et une sortie (6) pour l'écoulement d'un fluide (8) à travers la conduite (2) depuis l'entrée (4) jusqu'à la sortie (6), et ayant un tronçon de test (20) dans lequel le stent (18) peut être placé et qui peut être dilaté par une fluctuation de pression (Δp) sous forme d'une augmentation de la pression (p) du fluide (8),
**caractérisé par**
- une installation de fourniture (14) pour le fluide (8), pour fournir le fluide (8) à l'entrée (4) à un débit constant dans l'entrée (4) de la conduite (2), et
- un élément d'étranglement (16) pour le fluide (8), de façon à étrangler le flux en volume du fluide (8) qui sort de la sortie (6) selon des degrés différents de façon dynamique en fonction du temps, spécialement de façon périodique, de façon à créer, par les modifications de l'étranglement, des fluctuations dynamiques de pression (Δp) du fluide (8) en amont dans la conduite (2).

8. Agencement de conduite selon la revendication 7, dans lequel l'installation de fourniture (14) est une pompe (22), en particulier une pompe à engrenage, conçue pour introduire un fluide (8) dans l'entrée (4) selon un flux à volume constant, et/ou l'élément d'étranglement (16) est un modulateur de pression (24) connecté en série en aval de la sortie (6) et ayant un orifice de sortie (26), qui peut être connecté à une conduite (2) à travers laquelle le fluide (8) s'écoule de telle sorte que l'orifice de sortie forme l'extrémité de sortie pour le fluide (8), dans lequel l'orifice de sortie (26) débouche dans une chambre (28), de telle sorte que le fluide (8) pendant le fonctionnement forme un jet de fluide (30) sortant de l'orifice de sortie (26) et pénétrant dans la chambre (28) le long d'un chemin de fluide (32), et comportant un corps d'impact (34) qui peut être introduit dans le chemin de fluide (32) de manière variable, de façon dynamique en fonction du temps, formant au moins pendant une partie du temps une surface d'impact (36,36a-d), que vient heurter au moins une partie du jet de fluide (30).

9. Agencement de conduite (12) selon la revendication 8, dans lequel on peut modifier l'espace (a,a_{1,2}) et/ou l'angle d'inclinaison (α) de la surface d'impact par rapport à l'ouverture de sortie (26) et/ou le degré de recouvrement (G) entre la zone de section transversale (Q) du chemin de fluide (32) et la projection de la surface d'impact (36,36a-d) sur la zone de section transversale (Q).

10. Agencement de conduite (12) selon l'une des revendications 8 à 9, dans lequel le corps d'impact (34) et le chemin de fluide (32) peuvent être ajustés l'un par rapport à l'autre de telle sorte que, chaque fois qu'ils sont ajustés, des parties différentes de la surface du corps d'impact (34) forment des surfaces d'impact (36,36a-d) qui font saillie de façon variable dans le chemin de fluide (32) en fonction de l'ajustement entre les différentes positions relatives (R₁₋₄).

11. Agencement de conduite (12) selon la revendication 10, dans lequel le corps d'impact (34) et le chemin de fluide (32) sont déplaçables en rotation l'un par rapport à l'autre autour d'un axe de rotation (46), en particulier le corps d'impact (34) est déplaçable en rotation.

12. Agencement de conduite (12) selon la revendication 11, dans lequel le chemin de fluide (32) peut être déplacé en rotation par rapport au corps d'impact (34) de façon excentrique autour d'un axe de rotation (64) s'étendant en particulier parallèlement à la direction d'écoulement du chemin de fluide (32).

13. Agencement de conduite (12) selon l'une quelconque des revendications 10 à 12, dans lequel le corps d'impact (34) et le chemin de fluide (32), en particulier l'orifice de sortie (26) définissant le chemin de fluide (32), peuvent être déplacés en translation l'un par rapport à l'autre.

14. Agencement de conduite (12) selon l'une quelconque des revendications 8 à 13, dans lequel le corps d'impact (34) comprend une topographie de surface, en particulier ayant un profil de hauteur et/ou des interruptions (52), qui est distribuée sur le corps d'impact (34) de telle sorte que lorsque le corps d'impact (34) est introduit de façon variable dans le chemin de fluide (32), différents tronçons de la topographie de surface se tiennent de façon alternée dans le chemin de fluide (32).
